# DEMANDE DE BREVET EUROPEEN

(11) **EP 1 600 501 A1**
(43) Date de publication de la demande: **30.11.2005**
(21) Numéro de dépôt: 05290721.9
(22) Date de dépôt: 01.04.2005
(51) Int. Cl.: C12N 5/06

(54) **Utilisation du lif en ingénierie cellulaire et tissulaire**

(30) Priorité: 26.05.2004 FR 0451037
(71) Demandeur: L'OREAL, 75008 Paris (FR)
(72) Inventeur: Fortunel, Nicolas, 91650 Breuillet (FR); Bernard, Dominique, 75015 Paris (FR); Ferraris, Corinne, 75017 Paris (FR); Regnier, Marcelle, 75018 Paris (FR)
(74) Mandataire: Allab, Myriam

(57) **Abrégé**

L'invention concerne l'utilisation d'une quantité efficace d'un composé choisi parmi le Leukemia Inhibitory Factor ou facteur inhibiteur de leucémie (LIF), un analogue du LIF, un mimétique de LIF, un produit capable de stimuler l'expression du LIF endogène et leurs mélanges pour (i) favoriser la multiplication *in vitro* d'une population de cellules souches cutanées et/ou de progéniteurs épidermiques humains en les maintenant dans un état indifférencié et/ou pour (ii) maintenir et/ou augmenter leur capacité à générer un épithélium pluristratifié, en particulier un épiderme pluristratifié et/ou tout ou partie des annexes cutanées.

Elle porte également sur un procédé d'obtention d'une banque ou d'une culture de cellules souches cutanées et/ou de progéniteurs épidermiques humains indifférenciés en présence de LIF, un procédé d'obtention d'épidermes reconstruits et/ou de peaux reconstruites, des kits de production de banques de cellules ou d'épidermes reconstruits, et les utilisations du LIF notamment pour la préparation de cellules souches ou d'épidermes reconstruits destinés au traitement de sujets présentant une peau lésée (grands brûlés et sujets atteints de maladies génétiques affectant la peau).

## Description

La présente invention a pour domaine technique des procédés de culture de cellules souches cutanées et/ou de progéniteurs épidermiques humains et leurs utilisations notamment pour la préparation d'épithéliums pluristratifiés, en particulier d'équivalents d'épiderme et/ou de peau.

Elle a notamment pour objet l'utilisation d'une quantité efficace d'un composé choisi parmi le LIF, un analogue du LIF, un mimétique du LIF, un produit capable de stimuler l'expression endogène du LIF et leurs mélanges pour (i) favoriser la multiplication *in vitro* d'une population de cellules souches cutanées et/ou de progéniteurs épidermiques humains en les maintenant dans un état indifférencié et/ou pour (ii) maintenir et/ou augmenter leur capacité à générer un épithélium pluristratifié, en particulier un épiderme pluristratifié et/ou tout ou partie des annexes cutanées.

Elle porte également sur un procédé d'obtention d'une banque ou d'une culture de cellules souches cutanées et/ou de progéniteurs épidermiques humains indifférenciés en présence de LIF, un procédé d'obtention d'épidermes reconstruits et/ou de peaux reconstruites, des kits de production de banques de cellules ou d'épidermes reconstruits, et les utilisations du LIF notamment pour la préparation de cellules souches ou d'épidermes reconstruits destinés au traitement de sujets présentant une peau lésée (grands brûlés et sujets atteints de maladies génétiques affectant la peau).

Par 'population de cellules souches cutanées et/ou de progéniteurs épidermiques indifférenciés humains' selon l'invention, on entend une population de cellules souches somatiques adultes naturellement présentes dans la couche basale de l'épiderme, qui sont capables de s'autorenouveler et/ou proliférer et de générer un épithélium pluristratifié, en particulier un épiderme pluristratifié et/ou tout ou partie des annexes cutanées (glandes sébacées, follicule pileux, ongle...). On pourra par exemple obtenir cette population de cellules souches cutanées et/ou de progéniteurs épidermiques indifférenciés humains à partir de prélèvements de peau adulte et/ou de peau néonatale.

Par 'capacité d'une cellule à s'autorenouveler, on entend une cellule capable de se diviser pour donner deux cellules filles dont une au moins est identique à la cellule mère. A l'échelle d'une population cellulaire complexe, la notion d'autorenouvellement implique le maintien d'un compartiment de cellules de caractéristiques phénotypiques et fonctionnelles constantes au cours des divisions cellulaires successives. Selon l'invention, il s'agit du maintien d'un compartiment composé de cellules souches cutanées et/ou de progéniteurs épidermiques indifférenciés, notamment caractérisés par un fort potentiel de prolifération et une capacité à générer un épithélium pluristratifié, en particulier un épiderme pluristratifié.

Par 'capacité d'une cellule à proliférer', on entend une cellule capable de se multiplier pour donner deux cellules filles, sans qu'il y ait nécessairement transmission des caractéristiques et du potentiel de la cellule mère à l'une au moins des deux cellules filles.
La prolifération, qui peut ou non être associée au phénomène d'autorenouvellement, est susceptible de conduire à la diminution progressive ou la disparition du compartiment cellulaire d'intérêt au sein de la population cellulaire qui se multiplie.
Selon l'invention, la prolifération cellulaire est susceptible de s'accompagner d'une diminution progressive et/ou de la disparition du compartiment des cellules souches cutanées et/ou des progéniteurs épidermiques indifférenciés, notamment caractérisés par un fort potentiel de prolifération et une capacité à générer un épithélium pluristratifié, en particulier un épiderme pluristratifié.

Cette population comprend notamment des cellules souches cutanées et/ou des progéniteurs épidermiques indifférenciés.
Le compartiment des cellules souches, situé le plus en amont de la hiérarchie du tissu cutané, est caractérisé par un fort potentiel d'autorenouvellement (capacité de multiplication à long-terme la plus importante), ainsi que par une multipotentialité (notamment capacité à produire un épithélium pluristratifié, en particulier des kératinocytes et/ou différents types cellulaires des annexes cutanées, telles que follicules pileux, glandes sébacées, ongles...).
Les progéniteurs épidermiques, issus desdites cellules souches, présentent un potentiel de multiplication qui peut également être important, mais généralement plus réduit que celui des cellules souches, et sont caractérisés par une capacité à générer un épithélium pluristratifié, en particulier un épiderme pluristratifié.

Les cellules souches d'origine embryonnaire (ES) possèdent un potentiel d'expansion *a priori* illimité (Amit *et al*., *Dev Biol*. 227: 271-278, 2000; Odorico *et al*., *Stem Cells*. 19: 193-204, 2001). En effet, elles peuvent être multipliées sans signes de vieillissement tout en conservant un état indifférencié.

La demande WO 02/097068 décrit notamment une méthode d'induction de la différenciation de cellules souches murines embryonnaires en kératinocytes murins.

En revanche, le potentiel d'expansion des cellules souches somatiques adultes est limité par une horloge mitotique (Vaziri *et al*., *Proc Natl Acad Sci USA.* 91: 9857-9860, 1994 ; Chang *et al*., *Proc Natl Acad Sci USA.* 92: 11190-11194, 1995). Cette capacité d'expansion limitée concerne notamment les cellules souches et progéniteurs épidermiques (Ramirez *et al*., *Genes Dev*. 15: 398-403, 2 001 ; C ounter *et al*., *Lancet.* 361: 1345-1346, 2003 ; Fortunel *et al*., *J Cell Sci*. 116: 4043-4052, 2003).

Après un nombre limité de divisions, les cellules souches et les progéniteurs issus de tissus adultes s'orientent invariablement vers la différenciation, présentent des signes de sénescence, et meurent. Ces caractéristiques représentent des facteurs limitatifs pour la réalisation de banques de cellules et pour l'ingénierie cellulaire et tissulaire (ex : préparation d'équivalents de peau).

La Demanderesse avait précédemment proposé dans la demande WO 03/038073 (L'OREAUCNRS) un procédé d'enrichissement d'une population de cellules souches kératinocytaires à partir d'une préparation de kératinocytes, par adhésion de la préparation sur un composant de matrice extracellulaire.

Les méthodes de culture cellulaire, en particulier de multiplication et/ou de différenciation des cellules doivent donc tenir compte des caractéristiques propres aux cellules souches somatiques adultes et aux cellules souches embryonnaires, qui se distinguent notamment par (i) leur potentiel d'expansion, reconnu comme illimité pour les cellules souches embryonnaires et limité pour les cellules souches adultes ; et (ii) leur stade de différenciation, plus avancé pour les cellules souches cutanées et/ou progéniteurs épidermiques (aptes à générer les tissus cutanés), par rapport aux cellules souches embryonnaires dites 'totipotentes' (aptes à générer tous les types de tissus et organes de l'individu).

Il existe donc un besoin de trouver des agents capables de favoriser la multiplication des cellules souches cutanées et/ou des progéniteurs épidermiques humains en les maintenant dans un état indifférencié et qui soient capables de maintenir, voire augmenter leur potentiel organogénique, c'est-à-dire leur capacité à générer un épithélium pluristratifié, en particulier un épiderme pluristratifié et/ou tout ou partie des annexes cutanées (follicule pileux, glandes sébacées, ongles...).

Or la Demanderesse vient de découvrir de façon inattendue que l'utilisation du Leukemia Inhibitory Factor ou facteur inhibiteur de leucémie (LIF) permet :
(i) de favoriser la multiplication *in vitro* d'une population de cellules souches cutanées et/ou de progéniteurs épidermiques humains en les maintenant dans un état indifférencié ;
(ii) et apparaît même nécessaire pour l'obtention d'un épiderme reconstruit pluristratifié de qualité, c'est-à-dire présentant des caractéristiques histologiques comparables à celles d'un épiderme natif (avec une couche cornée, une couche granuleuse, des couches suprabasales et une couche basale). Elle a en effet montré qu'un épiderme reconstruit en l'absence de LIF présentait des anomalies de structures par rapport à un épiderme reconstruit en présence de LIF.

Le LIF est décrit dans le brevet US 6,261,548 comme un facteur capable de supprimer la prolifération de cellules myéloïdes leucémiques telles que les cellules murines M1, et à favoriser la différenciation des macrophages, suggérant son utilisation comme agent thérapeutique non prolifératif pour supprimer certaines formes de leucémies myéloïdes et pour modifier la fonction des macrophages impliqués dans la réponse aux infections.
Au niveau de la peau, le LIF est une cytokine naturellement produite par les kératinocytes *in vivo* et *in vitro* (Paglia *et al*., *Br J Dermatol*. 134: 817-823, 1996), et l'on sait que ce facteur est notamment impliqué dans le contrôle de processus inflammatoires associés à différentes pathologies cutanées comme le psoriasis (Bonifati *et al*., *Arch Dermatol Res.* 290: 9-13, 1998; Szepietowski *et al*., *J Dermatol*. 28: 115-122, 2001), ou certaines allergies (Szepietowski *et al*., *Contact Dermatitis.* 36: 21-25, 1997).

Cependant, il n'est nullement décrit ni suggéré une utilisation du LIF, d'un analogue du LIF, d'un mimétique du LIF, d'un produit capable de stimuler l'expression du LIF endogène ou leurs mélanges pour favoriser la multiplication *in vitro* de cellules souches somatiques adultes, connues pour avoir un potentiel d'expansion limité par rapport aux cellules souches embryonnaires, et un stade de différenciation plus avancé que ces dernières ; *a fortiori* l'utilisation du LIF pour favoriser la multiplication *in vitro* de cellules souches cutanées et/ou de progéniteurs épidermiques humains indifférenciés, ni pour améliorer les modèles de cultures organotypiques de peau (épidermes reconstruits et peaux reconstruites).

On comprend l'importance de cette découverte pour l'obtention de banques ou de cultures de cellules souches cutanées et/ou de progéniteurs épidermiques humains indifférenciés, et pour la préparation d'équivalents de peau destinés à traiter les sujets présentant une peau lésée (grands brûlés, sujets atteints d'une maladie génétique affectant la peau).

La peau constitue une barrière physique entre l'organisme et son environnement. Elle est constituée de deux tissus : l'épiderme et le derme.

Le derme fournit à l'épiderme un support solide. C'est également son élément nourricier. Il est principalement constitué de fibroblastes et d'une matrice extracellulaire elle-même notamment composée de collagène, d'élastine, de fibronectine, et d'une substance dite substance fondamentale, ces composants étant essentiellement synthétisés par les fibroblastes. On y trouve aussi des leucocytes, des mastocytes ou encore des macrophages tissulaires. Il contient également de vaisseaux sanguins et des fibres nerveuses.

L'épiderme est un épithélium pluristratifié desquamant, de 100 µm d'épaisseur en moyenne et est conventionnellement divisé en une couche basale contenant des cellules souches cutanées, des progéniteurs épidermiques indifférenciés, ainsi que des cellules engagées dans le processus de maturation/ différenciation, une couche dite épineuse constituée de plusieurs couches de cellules polyédriques disposées sur les cellules basales, une couche dite granuleuse constituée de cellules aplaties contenant des inclusions cytoplasmiques, les grains de kératohyaline, et enfin une couche supérieure appelée couche cornée (ou *stratum corneum*), constituée de kératinocytes au stade terminal de leur différenciation, appelés cornéocytes. Ces derniers sont des cellules kératinisées momifiées, anucléées qui dérivent des kératinocytes. L'empilement des cornéocytes constitue la couche cornée qui assure entre autres la fonction de barrière de l'épiderme.

La différenciation épidermique suit un processus de maturation continu et orienté dans lequel les kératinocytes basaux se transforment en migrant pour aboutir à la formation de cornéocytes, cellules mortes totalement kératinisées. Cette différenciation est la résultante de phénomènes parfaitement coordonnés qui vont conduire au maintien d'une épaisseur constante et assurer ainsi l'homéostasie de l'épiderme. Celle-ci implique une régulation précise du nombre de cellules qui entrent dans le processus de différenciation et du nombre de cellules qui desquament. Au cours du processus normal de desquamation, seuls les cornéocytes les plus superficiels se détachent de la surface de l'épiderme.

Du fait de son exposition, la peau peut être soumise à différents types d'agressions environnementales, dont les plus graves peuvent conduire à une issue fatale. Des patients ayant subi des brûlures sévères peuvent présenter une destruction du tissu cutané sur plus de 80% de la surface corporelle, et ne pourront être sauvés que si l'on parvient à produire une quantité d'équivalent de peau suffisante pour permettre la régénération de la peau sur l'ensemble des zones détruites.

La maîtrise du potentiel d'expansion des cellules souches cutanées et/ou de progéniteurs épidermiques et le développement de cultures organotypiques de peau à partir de cellules amplifiées *ex vivo* sont donc question d'importance majeure pour la production de greffons de tissu cutané autologue (Ronfard *et al*., *Burns*. 17: 181-184, 1991 et *Transplantation.* 70 : 1588-1598, 2000) et/ou hétérologue (Hefton *et al*., *Lancet.* 2: 428-430, 1983; Braye *et al*., *Med Biol Eng Comput*. 38: 248-252, 2000), destinés au traitement des grands brûlés.

Les équivalents d'épiderme produits à partir de cellules, issues d'explants de peau et amplifiées *ex vivo,* sont également utilisés pour la réalisation de greffes autologues permettant la régénération du tissu cutané, après excision chirurgicale d'un *nevus* (Kumagai *et al*., *Ann Plast Surg.* 39: 483-488, 1997), ou d'un tatouage (Kumagai *et al*., *An Plast Surg.* 33: 385-391, 1994).

La peau peut également être touchée par différentes maladies d'origine génétique, compromettant son intégrité. Il s'agit par exemple des épidermolyses bulbeuses, caractérisées par un défaut permanent de cohésion entre le derme et l'épiderme (Eady *et al*., *J Dermatol*. 28: 638-640, 2001); des ichtyoses, associées à un épaississement pathologique de la couche cornée de l'épiderme (DiGiovanna & Robinson-Bostom, *Am J Clin Dermatol*. 4: 81-95, 2003).

D'autres types de maladies génétiques touchant la peau, comme les *xeroderma pigmentosum,* conduisent à une altération de la capacité de ce tissu à répondre aux 'agressions' issues de l'environnement. Ces maladies se caractérisent par une déficience des mécanismes d'excision des lésions de l'ADN induites par les rayons ultra-violets (UV), avec pour conséquence clinique une augmentation de la fréquence d'apparition des cancers cutanés (Sarasin, *Mutat Res.* 428: 5-10, 1999).

Il s'agit donc de pathologies sévères, pour lesquelles aucun traitement pharmacologique classique efficace n'est actuellement disponible. Cependant, l'origine monogénique identifiée de certaines de ces maladies ouvre la perspective de traitements par thérapie génique (Spirito *et al*., *J Gen Med.* 3: 21-31, 2001 ; Magnaldo & Sarasin, *Mutat Res.* 509: 211-220, 2002).

En effet, une restoration de la fonction défective a été obtenue sur des kératinocytes issus d e prélèvements cutanés effectués chez des sujets malades, cultivés *ex vivo* et corrigés par transfert de gène à l'aide d'un vecteur rétroviral, dans le cas de plusieurs maladies génétiques de la peau. Par ce type d'approche, il a notamment été possible d'obtenir une correction génétique de kératinocytes atteints d'ichtyose lamellaire (Choate *et al*., *Hum Gene Ther.* 7: 2247-2253, 1996), d'ichtyose liée au chromosome X (Freiberg *et al*., *Hum Mol Genet.* 6: 927-933, 1997), ainsi que de pour différents groupes de *Xeroderma pigmentosum* (Carreau *et al*., *Hum Gene Ther.* 6: 1307-1315, 1995 ; Arnaudeau-Begard *et al*., *Hum Gene Ther.* 14: 983-996, 2003).

On comprend alors l'importance de disposer de systèmes de culture permettant de 'manipuler' *ex vivo* des cellules souches cutanées et/ou des progéniteurs épidermiques issus de sujets sains et/ou de patients atteints de maladies génétiques touchant la peau, ainsi que celle d'obtenir des équivalents d'épiderme et/ou de peau aussi physiologiques que possible à partir de ces cellules.

Un premier objet de l'invention est donc l'utilisation d'une quantité efficace d'un composé choisi parmi le Leukemia Inhibitory Factor (LIF), un analogue du LIF, un mimétique du LIF, un produit capable de stimuler l'expression du LIF endogène et leurs mélanges pour (i) favoriser la multiplication *in vitro* d'une population de cellules souches cutanées et/ou de progéniteurs épidermiques humains en les maintenant dans un état indifférencié et/ou pour (ii) maintenir et/ou augmenter leur capacité à générer un épithélium pluristratifié.

En particulier, l'invention concerne l'utilisation d'une quantité efficace d'un composé choisi parmi le LIF, un analogue du LIF, un mimétique du LIF, un produit capable de stimuler l'expression du LIF endogène et leurs mélanges dans un procédé destiné à l'obtention de banques ou de cultures de cellules souches cutanées et/ou de progéniteurs épidermiques humains indifférenciés.

Elle porte également sur l'utilisation d'une quantité efficace d'un composé choisi parmi le LIF, un analogue du LIF, un mimétique du LIF, un produit capable de stimuler l'expression du LIF endogène et leurs mélanges dans un procédé destiné à l'obtention d'épithéliums pluristratifiés, en particulier d'épidermes reconstruits et/ou des peaux reconstruites.

On parlera dans le contexte de l'invention d'équivalent d'épiderme ou d'épiderme reconstruit, ainsi que d'équivalent de peau ou de peau reconstruite.

L'action du LIF, d'un analogue du LIF ou d'un mimétique du LIF sur la multiplication *in vitro* d'une population de cellules souches cutanées et/ou de progéniteurs épidermiques humains indifférenciés, et/ou leur différenciation et/ou le développement d'un épiderme pluristratifié pourra être directe ou indirecte.

Elle pourra être directe, par stimulation du potentiel de prolifération, d'expansion, et/ou d'organogenèse des cellules souches cutanées et/ou des progéniteurs épidermiques. Elle pourra également être indirecte, notamment par le biais de facteurs et/ou de signaux émis par les cellules réactives au LIF en réponse à ce facteur, et capables de stimuler d'une manière bénéfique ces mêmes cellules et/ou les cellules voisines. Il s'agit par exemple de signaux transmis par contact direct entre les cellules, et/ou de facteurs impliqués dans des voies de régulation autocrines et/ou paracrines. Le contrôle de l'expression du potentiel des cellules souches cutanées et/ou des progéniteurs épidermiques implique notamment ce type de 'dialogue' entre les fibroblastes du derme et les souches et/ou les progéniteurs épidermiques. Lesdites cellules seront dès lors plus réceptives à des stimuli exogènes et/ou à un signal issu de l'environnement des cellules.

L'homéostasie épidermique résulte notamment d'une balance finement régulée entre signaux mitogènes favorisant la division cellulaire et signaux anti-prolifératifs. Ces signaux résultent notamment de l'action des facteurs naturellement produits par les kératinocytes et/ou par les autres types cellulaires présents dans leur environnement, notamment sécrétés par les fibroblastes du derme.

Comme exemples de 'stimuli et/ou de signaux connus pour être impliqués dans l'homéostasie épidermique', on peut notamment citer :
- des facteurs de croissance, tels que les facteurs de croissance mitogéniques 'epidermal growth factor' (EGF) et 'keratinocyte growth factor' (KGF) (Cook *et al*., *J Cell Physiol*. 146: 277-289, 1991 ; Andreadis *et al*., *FASEB J.* 15: 898-906, 2001 ; Gamady *et al*., *J Cell Biochem.* 89: 440-449, 2003), ou encore le 'transforming growth factor-β1' (TGF-β1), facteur de croissance multifonctionnel notamment identifié pour son effet anti-prolifératif sur les kératinocytes *in vivo* et *in vitro* (Glick *et al*., *Proc Natl Acad Sci USA.* 90: 6076-6080, 1993 ; Van Ruissen *et al*., *J Cell Sci.* 107: 2219-2228, 1994; Cui *et al*., *Genes Dev*. 9: 945-955, 1995). Ces facteurs sont impliqués dans des boucles de régulation autocrines et paracrines fortement interactives et imbriquées, assurant à la fois le contrôle de la prolifération et celui de la différenciation (Reiss & Sartorelli, *Cancer Res.* 47: 6705-6709, 1987 ; Hertle *et al*., *J invest Dermatol*. 104: 260-265, 1995 ; Edmonson et *al*., *J Cell Physiol*. 179: 201-207, 1999 ; Yamasaki *et al*., *J Invest Dermatol*. 120: 1030-1037, 2003 ; Pasonen-Seppanen *et al*., *J Invest Dermatol*. 120: 1038-1044, 2003), et permettant ainsi une régulation précise du renouvellement et/ou de la maturation des kératinocytes ; et/ou
- des molécules pouvant compléter, moduler, et/ou interférer avec l'action des facteurs de croissance, telles que par exemple : la vitamine D et ses dérivés pour leur capacité à augmenter la sensibilité des kératinocytes à l'action mitogénique du KGF (Gamady *et al*., *J Cell Biochem.* 89: 440-449, 2003) ; l'acide rétinoïque pour sa capacité à moduler la prolifération et/ou la différenciation des kératinocytes (Choi & Fuchs, *Cell Regul*. 1: 791-809, 1990; Gibbs *et al*., *Arch Dermatol Res.* 288: 729-738, 1996 ; Chapellier *et al*., *EMBO J.* 21: 3402-3413, 2002), notamment par un mécanisme de sensibilisation des cellules à l'action de l'EGF et du TGF-β (Tong *et al*., *J Invest Dermatol*. 94: 126-131, 1990).

Le 'LIF' utilisé selon l'invention peut notamment être apporté sous la forme d'un LIF purifié, d'un LIF recombinant, d'un broyat ou extrait cellulaire contenant du LIF, d'un surnageant de culture de cellules contenant du LIF, ou leurs mélanges.
De préférence on utilisera un LIF d'origine humaine.

On peut notamment utiliser:
- un LIF purifié à partir d'organes, de tissus, et/ou de cellules exprimant naturellement le LIF [ex: cellules de glande pituitaire (Ferrara *et al*., *Proc Natl Acad Sci USA.* 89: 6 98-702, 1 992), fibroblastes d u derme (Lorenzo *et al*., *Clin Immunol Immunopathol*. 70: 260-265, 1994), fibroblastes pulmonaires (Elias *et al*., *Am J Physiol*. 266: L426-435, 1994), placenta et endomètre (Kojima *et al*., *Biol Reprod.* 50: 882-887, 1994), cellules stromales de moelle osseuse (Lorgeot *et al*., *Cytokine*. 9: 754-758, 1997), cellules de rein (Morel *et al*., *Cytokine.* 12: 265-271, 2000), cardiomyocytes (Ancey *et al*., *Cytokine.* 18: 199-205, 2002)] ;
- un LIF recombinant, tel qu'obtenu en culture de microorganismes prokaryotes (ex : bactérie, *E. Coli*) ou de cellules eucaryotes (ex : levure, *Pichia pastoris, Saccharomyces),* sous forme ou non d'une protéine de fusion (ex : LIF recombinant humain commercialisé par Chemicon International Inc.) ;
- un broyat ou un extrait de cellules exprimant le LIF, en particulier un broyat ou un extrait de cellules nourricières (ex : fibroblastes murins de la lignée 3T3) exprimant le LIF, ou de cellules génétiquement modifiées pour exprimer le LIF, ou encore de cellules stimulées pour exprimer le LIF ;
- un surnageant de culture de cellules contenant du LIF, tel qu'un surnageant de culture de cellules 3T3 exprimant le LIF ;
ou leurs mélanges.

Comme 'produit capable de stimuler l'expression du LIF endogène', on peut citer par exemple l'IL-1β, cytokine décrite pour sa capacité à induire la synthèse et la sécrétion de LIF par les fibroblastes, cellules épithéliales et cellules musculaires lisses du poumon humain (Knight *et al*., *Am J Respir Cell Mol Biol*. 20: 934-841, 1999). On peut également citer le TNF-α, ainsi que l'analoque d'AMP cyclique 8-bromoadénosine 3': 5' monophosphate (8BrcAMP), étudiés pour leur capacité à induire le promoteur du LIF dans une lignée murine de cellules issues du stroma médullaire (Gollner *et al*., *Cytokine.* 11: 656-663, 1999).
Selon une alternative, on peut utiliser tout principe actif capable au contraire de réprimer l'activité de voies de signalisation antagonistes de l'effet anti-différenciateur du LIF, et notamment celle impliquant la protéine Stat5. Il s'agit notamment d'oligonucléotides antisens, et/ou de méthodes d'interférence par des ARN de petite taille (siRNA), ou encore de modulateurs des activités kinases et phosphatases impliquées dans l'activation des molécules Stats.

Par 'analogue du LIF' selon l'invention, on entend notamment tout polypeptide LIF modifié ou tout fragment de polypeptide LIF ayant une activité LIF sur les cellules souches cutanées et/ou les progéniteurs épidermiques, c'est-à-dire qui est capable (i) de favoriser la capacité des cellules souches cutanées et/ou de progéniteurs épidermiques indifférenciés à s'autorenouveler et/ou à proliférer et (ii) de maintenir et/ou augmenter leur capacité à générer un épiderme pluristratifié.

Par 'polypeptide LIF modifié ayant une activité LIF', on entend notamment un polypeptide LIF ayant subi une ou plusieurs modifications, par exemple pour augmenter sa stabilité. Par 'modification', on entend toute substitution, délétion et/ou insertion d'un acide aminé ou d'un nombre réduit d'acides aminés, notamment par substitution d'acides aminés naturels par des acides aminés non naturels ou pseudoacides aminés à des positions telles que les modifications ne portent pas significativement atteinte à l'activité biologique du LIF.
Le polypeptide LIF modifié pourra être obtenu à partir d'une séquence peptidique de LIF humain choisie parmi la séquence Genbank AAA59217 (195 aa), la séquence GenBank AAA51699 (202 aa), et les séquences homologues.
Par 'séquence homologue', on entend une séquence identique à au moins 70%, de préférence au moins 85% et encore plus préférentiellement au moins 95% d'une séquence peptidique définie, chez la même espèce ou chez une espèce différente ; on parle alors de séquence peptidique orthologue.
On pourra également obtenir ce polypeptide LIF modifié à partir des séquences du gène ou de l'ADNc du LIF humain (GenBank M63420 J05436, J03261, X13967), des séquences du gène ou de l'ADNc du LIF murin (GenBank M63419 J05435, X06381, X12810, S73374), selon les techniques classiques de clonage et d'expression.

Par 'fragment de polypeptide LIF ayant une activité LIF', on entend notamment un fragment d'une séquence choisie parmi la séquence Genbank AAA59217 (195 aa), la séquence GenBank AAA51699 (202 aa) et les séquences homologues.
Ledit fragment aura notamment une taille suffisante pour reconstituer la structure tertiaire du LIF présentant les sites de liaison au LIF-R et à gp130.
Ce fragment de polypeptide pourra également être obtenu selon les techniques classiques de clonage et d'expression à partir des séquences du gène ou de l'ADNc du LIF humain (GenBank M63420, J05436, X13967, J03261), des séquences du gène ou de l'ADNc du LIF murin (GenBank X06381, M63419 J05435, X12810, S73374), et en particulier à partir des séquences codantes.

De tels analogues du LIF adaptés à la mise en oeuvre de l'invention pourront ainsi être sélectionnés selon le procédé comprenant les étapes suivantes :
a) on met en culture une préparation de kératinocytes ou de cellules souches cutanées et/ou de progéniteurs épidermiques indifférenciés (i) en présence ou (ii) en absence du produit à tester ;
b) on étudie au microscope les cellules cultivées selon (i) et (ii) ;
c) on mesure la capacité du produit à favoriser la multiplication *in vitro* des cellules souches cutanées et/ou des progéniteurs épidermiques indifférenciés en comparant le nombre de clones cellulaires à l'état indifférencié obtenus (i) en présence ou (ii) en absence du produit à tester ;
d) on sélectionne le produit pour lequel on obtient un nombre de clones cellulaires à l'état indifférencié augmenté en présence dudit produit par rapport au nombre de clones cellulaires à l'état indifférencié en absence dudit produit ;
e) on t este ensuite la capacité dudit produit à régénérer un épiderme p luristratifié selon les étapes suivantes :
   a. on ensemence sur un support de derme une préparation de kératinocytes (i) en présence ou (ii) en absence dudit produit à tester ;
   b. on observe au microscope la structure de l'épiderme reconstruit selon (i) et (ii) ;
   c. on sélectionne le produit pour lequel on obtient une structure de l'épiderme reconstruit améliorée en présence dudit produit par rapport à la structure de l'épiderme en absence dudit produit.

Par 'mimétique du LIF' selon l'invention, on entend notamment tout agoniste du récepteur du LIF (LIF-R) ou encore tout actif, extrait ou fraction d'extrait cellulaire capable d'activer la gp130 et/ou les voies de signalisation Jak/Stat et Ras/Map kinases, en particulier l'expression et/ou l'activité de Stat3.
On pourra par exemple utiliser des anticorps agonistes des récepteurs du LIF, des peptides de synthèse capables d'interagir avec les récepteurs du LIF et de les activer ou tout actif capable d'induire une activation des voies de signalisation impliquées dans la réponse au LIF, en particulier l'expression et/ou l'activité de Stat3, ou sur l'activation des Janus-associated tyrosine kinases (JAK).

En particulier, on pourra utiliser des agents capables d'induire la formation d'un hétérodimère LIFR-gp130, tels que par exemple la cytokine oncostatine M (OSM), le ciliary neurotrophic factor (CNTF) et la cardiotrophin-1 (CT-1), qui ont une forte homologie de structure tertiaire avec le LIF.
De tels mimétiques du LIF adaptés à la mise en oeuvre de l'invention pourront être sélectionnés par des tests classiques de liaison au LIF-R et d'activation de la gp130.

Ces analogues du LIF ou mimétiques du LIF pourront être d'origine naturelle ou synthétique.
Par 'origine naturelle', on entend un composé à l'état pur ou en solution à différentes concentrations, obtenu par différents procédés d'extraction à partir d'un tissu (peau...) d'origine naturelle, en particulier l'épiderme humain ou à partir d'extraits d'origine végétale.
Par 'origine synthétique', on entend un composé à l'état pur ou en solution à différentes concentrations, obtenu chimiquement ou par production dans un organisme après introduction dans cet organisme des éléments nécessaires à cette production.

On sait en effet que la réponse cellulaire au LIF implique un récepteur de faible affinité (glycoprotéine de 190 kD, gp 190) et un récepteur de forte affinité (glycoprotéine de 130 kD, gp 130) (Taupin *et al*., *J Biol Chem.* 276: 47975-47981, 2001), et en aval de ces récepteurs, les voies de signalisation Jak/Stat et Ras/Map kinases (Ernst *et al*., *J Biol Chem.* 274: 9729-9737, 1999 ; Burdon *et al*., *Trends Cell Biol*. 12: 432-438, 2002). La protéine de transduction du signal Stat3 joue un rôle prépondérant dans le maintien de l'état indifférencié des cellules ES murines en réponse au LIF (Niwa *et al*., *Genes Dev*. 12: 2048-2060, 1998; Matsuda *et al*., *EMBO J.* 18: 4261-4269, 1999), alors que l'expression de la protéine Stat5 est au contraire associée à un engagement de ces cellules vers la différenciation (Nemetz *et al*., *Differentiation.* 62: 213-220, 1998).

Selon une alternative, on pourra également associer à un LIF, un analogue du LIF, un mimétique du LIF ou un produit capable de stimuler l'expression du LIF, un récepteur du LIF (LIF-R) ou un produit capable de stimuler l'expression du LIF-R endogène.

L'invention porte également sur un procédé d'obtention d'une banque ou d'une culture de cellules souches cutanées et/ou de progéniteurs épidermiques indifférenciés, comprenant au moins une étape de multiplication d'une préparation de kératinocytes humains dans un milieu de culture contenant une quantité efficace d'un composé choisi parmi le LIF, un analogue du LIF,un mimétique du LIF, un produit capable de stimuler l'expression du LIF endogène et leurs mélanges.

Par 'culture de cellules' selon l'invention, on entend notamment une préparation de cellules issues d'un tissu natif, cultivées *in vitro* et maintenues vivantes dans des conditions artificielles *(ex vivo, in vitro*).

Par 'banque de cellules' selon l'invention, on entend notamment une préparation de cellules traitées pour être conservées en vue d'une utilisation ultérieure. Les cellules pourront par exemple être congelées et conservées sous la forme d'aliquots. Les cellules peuvent provenir de tissus natifs et être soit directement stockées, par exemple sous forme congelée, soit amplifiées *in vitro* avant d'être stockées.

Par 'préparation d e kératinocytes humains' selon l'invention, on entend notamment un ensemble de kératinocytes obtenus à partir d'un prélèvement cutané et/ou de toutes autres sources possibles de cellules souches cutanées et/ou de progéniteurs capables de générer de l'épiderme et/ou tout ou partie des annexes cutanées.
Comme source alternative desdites cellules souches et/ou progéniteurs, on peut citer notamment le follicule pileux.

Une préparation de kératinocytes peut être obtenue selon les méthodes classiques de culture cellulaire.
En particulier, on pourra, à partir d'un explant cutané prélevé sur un sujet, procéder comme suit:
- on élimine le tissu sous-cutané à l'aide d'un scalpel ;
- on décontamine le prélèvement cutané par un traitement antibiotique (ex : gentamycine) ;
- on sépare le derme de l'épiderme par traitement protéolytique (ex: trypsine et dispase) puis dissection ;
- on favorise ensuite la dissociation des cellules en présence d'une solution de trypsine 0,05% et EDTA 0,02% ; et on neutralise l'effet de la trypsine par l'ajout d'un milieu de culture DMEM contenant 10% de sérum;
- on homogénéise la suspension cellulaire, que l'on lave ensuite dans du milieu de culture pour kératinocytes (KGM, Bullet kit, Clonetics Corp).

L'étape de multiplication de la préparation de kératinocytes pourra être réalisée dans un milieu de culture contenant du LIF, un analogue du LIF, un mimétique du LIF ou un produit capable de stimuler l'expression du LIF endogène, adapté à la multiplication desdites cellules et à leur maintien dans un état indifférencié.
On pourra par exemple utiliser un milieu de culture semi-défini (KGM Bullet Kit, Clonetics, Cambrex B io Science Inc.), en absence de fibroblastes nourriciers, et à faible densité (par exemple ensemencement à 2400 cellules/cm²).
On pourra également utiliser le milieu décrit par Rheinwald et Green (*Cell*. 6: 317-330, 1975), en présence de fibroblastes irradiés, et à faible densité (par exemple ensemencement à 2400 cellules/cm²).

Plus généralement, tout milieu de culture comprenant du LIF et destiné à favoriser la multiplication des cellules souches cutanées et/ou des progéniteurs épidermiques et/ou pour générer un épiderme stratifié, fera également partie de l'invention.

En particulier, un tel milieu de culture pourra notamment contenir au moins un facteur de croissance mitogénique pour les kératinocytes [ex : epidermal growth factor (EGF) et/ou keratinocyte growth factor (KGF)], de l'insuline, de l'hydrocortisone et un antibiotique (ex : gentamycine, amphotericine B).
Avantageusement, ledit milieu pourra comprendre en outre un extrait pituitaire, par exemple d'origine bovine, de l'épinephrine, de la transferrine et/ou des acides aminés non essentiels.
Ledit milieu pourra contenir ou non du sérum et éventuellement être additionné du facteur de croissance 'transforming growth factor-β' (TGF-β).

La concentration efficace de LIF, analogue du LIF, mimétique du LIF, ou d'un produit capable de stimuler l'expression du LIF endogène ou leurs mélanges présente dans le milieu de culture desdites cellules pourra être comprise entre 0.01pg/ml et 1mg/ml de milieu de culture, préférentiellement entre 0.1ng/ml et 100ng/ml de milieu de culture, et encore plus préférentiellement entre 100pg/ml et 1ng/ml de milieu de culture.

Si l'on utilise un broyat ou un extrait cellulaire contenant du LIF, la concentration efficace dudit broyat ou extrait présente dans le milieu de culture de l'étape de multiplication desdites cellules pourra être comprise entre 0.01pg/ml et 10mg/ml de milieu de culture, préférentiellement entre 10pg/ml et 100µg/ml de milieu de culture, avantageusement entre 100ng/ml et 1 µg/ml de milieu de culture.

Selon un mode préféré, la préparation de kératinocytes humains est préalablement enrichie en cellules souches cutanées et/ou en progéniteurs épidermiques indifférenciés, en particulier par adhésion rapide sur un composant de matrice extracellulaire et récupération des cellules ayant adhéré (cellules à forte capacité d'adhésion, nommées Adh⁺⁺⁺).
Par 'composant de matrices extracellulaires', on désigne notamment des molécules telles que les collagènes, les laminines, la fibronectine, les protéoglycanes.
On peut notamment utiliser l'étape de pré-enrichissement décrite dans la demande WO03/038073 (L'OREAUCNRS).
De préférence, l'étape d'adhésion est réalisée sur un support plastique sur lequel du collagène de type I est adsorbé ; et cette étape est réalisée à 37°C pendant une durée d'environ 10mn à environ 20mn, en particulier de 12 à 15mn.
Et l'étape de décollement peut être réalisée par trypsinisation douce, par exemple en présence de trypsine 0.05% et EDTA 0.02% pendant une durée brève, de préférence inférieure à 10mn, en particulier pendant une durée de 1 à 2mn.

Selon un mode particulier, la suspension cellulaire est placée dans des flacons de culture « recouverts » avec du collagène de type I (solution de collagène I (Sigma Chemical Co Ltd, Irvine, UK) diluée d'un facteur 2 dans du PBS, déposée pendant 45 minutes dans les flacons, puis séchage après élimination du surplus), à une densité de 200 000 cellules/cm². Après 12 minutes, les kératinocytes n'ayant pas adhéré sont éliminés par lavage en tampon PBS. Les cellules adhérentes ainsi sélectionnées sont détachées du support par une trypsination douce (trypsine 0,05%-EDTA 0,02% (Biological Industries, Kibbutz Beit Haemek, Israël) pendant 3 à 5 minutes à 37°C). Après neutralisation de la trypsine (DMEM+10%SVF), les cellules sont récupérées, lavées, puis remises en suspension dans du milieu KGM.
La fraction des cellules adhérentes sélectionnée par cette méthode représente environ 10% des kératinocytes totaux de l'épiderme.

Le pourcentage de cellules adhérentes représente généralement 5 à 20% des cellules contenues dans la préparation de kératinocytes, en particulier 10% des cellules contenues dans la préparation de kératinocytes.
La population dite Adh⁺⁺⁺ ainsi sélectionnée présente une fréquence de progéniteurs épidermiques indifférenciés et/ou de cellules souches cutanées clonogéniques environ 10 fois supérieure à celle de la population de kératinocytes plus matures caractérisée par une plus faible capacité d'adhésion (Adh^{-/+}).

Le procédé d'enrichissement permet ainsi de sélectionner une population de cellules adhérentes à fort potentiel d'expansion par rapport aux cellules non adhérentes, majoritairement constituées de kératinocytes matures.
Ladite population de cellules adhérentes Adh⁺⁺⁺ comprend des cellules souches cutanées et/ou des progéniteurs épidermiques indifférenciés notamment caractérisés par un potentiel d'expansion à long terme, et un potentiel à générer un épithélium pluristratifié, notamment un épiderme pluristratifié, et/ou un équivalent de peau, et/ou tout ou partie des annexes cutanées.

Selon un mode particulier, un enrichissement de la préparation de kératinocytes et/ou de la population de cellules souches cutanées et/ou de progéniteurs épidermiques, en cellules réactives au LIF, c'est-à-dire en cellules présentant à leur surface une expression du ou des récepteurs du LIF (cellules de phénotype LIF-R⁺) et/ou en cellules capables de se multiplier dans un état indifférencié en présence de LIF, pourra également être réalisé. La sélection des cellules LIF-R⁺ réactives au LIF pourra par exemple être réalisée par marquage immuno-phénotypique et tri de cellules viables par cytométrie en flux, à l'aide de billes immuno-magnétiques, et/ou par immuno-sélection sur un support ou sont adsorbés des anticorps anti-LIF-R.

Avantageusement, le procédé selon l'invention comprend en outre une étape de cryo-conservation desdites cellules multipliées, dans des conditions favorisant leur maintien dans un état différencié.
Cette étape de cryo-conservation, utilisée notamment pour l'obtention de banques de cellules, consiste à congeler les cellules en azote liquide après expansion en présence de LIF, afin de permettre le stockage et/ou la conservation desdites cellules en vue d'une utilisation ultérieure.

Ce procédé de multiplication *in vitro* en présence de LIF selon l'invention permet ainsi l'obtention d'une banque ou d'une culture de cellules souches cutanées et/ou de progéniteurs épidermiques indifférenciés.

Cette banque ou culture de cellules souches cutanées et/ou de progéniteurs épidermiques indifférenciés est notamment caractérisée en ce qu'elle est enrichie en cellules capables de répondre à l'action du LIF et/ou exprimant à leur surface des récepteurs du LIF (LIF-R), nommées LIF-R⁺.
Elle peut se présenter sous forme liquide ou avantageusement sous forme congelée.

Tout kit de production d'une banque ou d'une culture de cellules souches cutanées et/ou de progéniteurs épidermiques humains indifférenciés comprenant au moins (i) une préparation de kératinocytes humains et (ii) un milieu de culture de cellules kératinocytaires humaines additionné d'une quantité efficace de LIF, d'un analogue du LIF ou d'un mimétique du LIF fera également partie de l'invention.
En particulier, ledit kit comprendra un milieu dans lequel la quantité efficace de LIF est comprise entre 0.01pg/ml et 1mg/ml de milieu de culture, préférentiellement entre 0.1 et 100ng/ml de milieu de culture.
Si l'on utilise un broyat ou un extrait cellulaire contenant du LIF, la concentration efficace dudit broyat ou extrait présente dans le milieu de culture pourra être comprise entre 0.01pg/ml et 10mg/ml de milieu de culture, préférentiellement entre 10pg/ml et 100µg/ml de milieu de culture, avantageusement entre 100ng/ml et 1µg/ml de milieu de culture.

L'invention porte également sur un procédé de préparation d'épidermes reconstruits et/ou de peaux reconstruites comprenant :
a) une étape de préparation d'un support ou d'un équivalent de derme ; et
b) une étape d'ensemencement d'une population de k ératinocytes humains sur ledit support ;
une quantité efficace d'un composé choisi parmi le LIF, un analogue du LIF, un mimétique du LIF et un produit capable de stimuler l'expression du LIF endogène étant ajoutée dans le milieu, à l'une des étapes a) ou b), ou les deux.

Selon une alternative, le procédé de préparation d'épidermes reconstruits et/ou de peaux reconstruites comprend :
a) une étape de préparation d'un support ou d'un équivalent de derme; et
b) une étape d'ensemencement d'une population de cellules souches cutanées et/ou de progéniteurs épidermiques indifférenciés cultivés en présence d'un composé choisi parmi le LIF, un analogue du LIF, un mimétique du LIF, un produit capable de stimuler l'expression du LIF endogène et leurs mélanges, sur ledit support.

En particulier, ledit support ou équivalent de derme sera choisi parmi des lattices de collagène/ fibroblastes, un derme préalablement désépidermisé, des membranes artificielles.

On peut citer à titre d'exemple de préparation de derme équivalent les protocoles décrits dans les demandes de brevets (EP-A-285471, EP-A-285474, EP-A-789074, EP-A-502172, EP-A-418035, WO-A-9116010, EP-A-197090, EP-A-20753, FR-A-2665175, FR-A-2689904) ou de préférence le protocole décrit par Asselineau *et al*., 1987, (Models in dermato., vol.III, Ed. Lowe & Maibach, 1-7).

On peut citer comme exemple de protocoles de préparation d'équivalents d'épiderme et/ou de peau, ceux décrits dans les brevets ou dans les demandes de brevets EP 285471, EP 285474, EP 418035, WO-A-90 02796, WO-A-9116010, EP 197090, EP 20753, FR 2665175, FR 2689904.
De manière très générale, les modèles de peau reconstruite sont constitués de kératinocytes humains déposés sur un support, souvent un équivalent de derme, et
cultivés dans des conditions telles qu'ils entrent dans un programme de différenciation aboutissant à la formation d'un équivalent d'épiderme.
On peut également intégrer d'autres types cellulaires tels que les cellules de Langherans (EP0789074) ou les mélanocytes, pour reconstituer un épiderme et/ou une peau proches des tissus natifs.

Tout kit de production d'équivalents d'épiderme et/ou d'équivalents de peau comprenant au moins une quantité efficace d'un composé choisi parmi le LIF, un analogue du LIF,un mimétique du LIF, un produit capable de stimuler l'expression du LIF endogène et leurs mélanges, fait également partie de l'invention.
En particulier, ce kit pourra comprendre (i) un support ou équivalent de derme, (ii) une préparation de kératinocytes et (iii) une quantité efficace d'un composé choisi parmi le LIF, un analogue du LIF, un mimétique du LIF, un produit capable de stimuler l'expression du LIF endogène et leurs mélanges.
Le LIF, analogue du LIF, mimétique du LIF, un produit capable de stimuler l'expression du LIF endogène et leurs mélanges pourra être apporté seul ou en mélange dans un milieu de culture, ledit milieu de culture étant adapté à la multiplication et/ou à la différenciation des kératinocytes.
Comme décrit ci-dessus, un tel milieu pourra notamment contenir au moins un facteur de croissance mitogénique pour les kératinocytes [ex : epidermal growth factor (EGF) et/ou keratinocyte growth factor (KGF)], de l'insuline, de l'hydrocortisone et un antibiotique (ex : gentamycine, amphotericine B).
Avantageusement, ledit milieu pourra comprendre en outre un extrait pituitaire, par exemple d'origine bovine, de l'épinephrine, de la transferrine et/ou des acides aminés non essentiels.

Ledit milieu pourra contenir ou non du sérum et éventuellement être additionné du facteur de croissance 'transforming growth factor-β' (TGF-β).

Un autre aspect de l'invention porte sur les multiples utilisations du LIF tel que défini selon l'invention et des cellules souches cutanées et/ou de progéniteurs épidermiques indifférenciés amplifiés en présence de LIF.

En particulier, l'invention concerne l'utilisation de cellules souches cutanées et/ou de progéniteurs épidermiques indifférenciés amplifiés en présence d'un composé choisi parmi le LIF, un analogue du LIF, un mimétique du LIF, un produit capable de stimuler l'expression du LIF endogène et leurs mélanges dans un procédé de criblage et/ou d'évaluation d'actifs susceptibles de moduler la mutiplication et/ou la différenciation de cellules souches cutanées et/ou de progéniteurs épidermiques indifférenciés en kératinocytes matures, ou en tout autre type cellulaire entrant dans la constitution des annexes cutanées.

En particulier, ce procédé de criblage comprend :
- une mise en culture de cellules souches cutanées et/ou de progéniteurs épidermiques indifférenciés amplifiés en présence d'un composé choisi parmi le LIF, un analogue du LIF,un mimétique du LIF, un produit capable de stimuler l'expression du LIF endogène et leurs mélanges, dans des conditions permettant leur multiplication et/ou leur différenciation en kératinocytes matures ou en tout autre type cellulaire entrant dans la constitution des annexes cutanées, et en présence d'un actif à tester ;
- la comparaison de la multiplication et/ou de la différenciation des cellules en présence de l'actif avec la multiplication et/ou la différenciation des cellules en l'absence dudit actif.

L'invention porte également sur l'utilisation de cellules souches cutanées et/ou de progéniteurs épidermiques indifférenciés amplifiés en présence d'un composé choisi parmi le LIF, un analogue du LIF, un mimétique du LIF, un produit capable de stimuler l'expression du LIF endogène et leurs mélanges dans un procédé de criblage et/ou d'évaluation d'actifs susceptibles de moduler la capacité des cellules souches cutanées et/ou des progéniteurs épidermiques à générer un épithélium pluristratifié, en particulier un équivalent d'épiderme et/ou un équivalent de peau.

En particulier, ce procédé comprend :
- une mise en culture de cellules souches cutanées et/ou de progéniteurs épidermiques indifférenciés amplifiés en présence d'un composé choisi parmi le LIF, un analogue du LIF, u n mimétique d u LIF, un produit capable de stimuler l'expression du LIF endogène et leurs mélanges, dans des conditions leur permettant de générer un épiderme pluristratifié et/ou tout ou partie des annexes cutanées, en présence d'un actif à tester ;
- la comparaison de la capacité des cellules à générer un épiderme pluristratifié et/ou tout ou partie des annexes cutanées en présence de l'actif avec la capacité des cellules à générer un épiderme pluristratifié et/ou tout ou partie des annexes cutanées en l'absence dudit actif.

Un autre volet de l'invention est l'utilisation d'un composé choisi parmi le LIF, un analogue du LIF, un mimétique du LIF et un produit capable de stimuler l'expression du LIF endogène pour la préparation de cellules souches cutanées et/ou de progéniteurs épidermiques indifférenciés ou d'épidermes reconstruits et/ou de peaux reconstruites destinés à traiter des lésions de la peau.

En particulier, les cellules souches cutanées et/ou les progéniteurs épidermiques indifférenciés ou les épidermes reconstruits et/ou les peaux reconstruites sont destinés à régénérer *in vivo* une peau saine sur une peau lésée ou des zones de peau lésée.

Les causes de lésions de la peau peuvent être multiples :
- des causes accidentelles, par exemple une peau ayant été brûlée sur une grande surface du corps ou du visage (ex : grands brûlés) ;
- des causes chirurgicales, par exemple une peau ayant subi une excision pour le traitement d'un nevus (Kumagai *et al*., *Ann Plast Surg.* 39: 483-488, 1997), ou d'un tatouage (Kumagai *et al*., *An Plast Surg.* 33 : 385-391, 1994). ;
- des causes génétiques, par exemple une peau présentant des altérations liées à une maladie génétique ; on peut citer notamment les épidermolyses bulbeuses, caractérisées par un défaut permanent de cohésion entre le derme et l'épiderme (Eady *et al*., *J Dermatol*. 28: 638-640, 2001); des ichtyoses, associées à un épaississement pathologique de la couche cornée de l'épiderme, telles que l'ichtyose lamellaire (Choate *et al*., *Hum Gene Ther.* 7: 2247-2253, 1996), l'ichtyose liée au chromosome X (Freiberg *et al*., *Hum Mol Genet.* 6: 927-933, 1997) ; des *Xeroderma pigmentosum,* qui se caractérisent par une déficience des mécanismes d'excision des lésions de l'ADN induites par les rayons ultra-violets (UV), avec pour conséquence clinique une augmentation de la fréquence d'apparition des cancers cutanés (Sarasin, *Mutat Res.* 428: 5-10, 1999).

Le traitement d'une peau lésée, dans les cas où la peau est lésée de façon accidentelle ou suite à une intervention chirurgicale, pourra consister en :
- la préparation d'une population de cellules souches cutanées et/ou de progéniteurs épidermiques humains indifférenciés à partir d'un explant cutané de sujet sain (cellules hétérologues) ou de zones saines du sujet présentant une peau lésée (cellules autologues);
- la multiplication de ladite population de cellules souches cutanées et/ou de progéniteurs épidermiques humains indifférenciés en présence de LIF;
- la ré-implantation desdites cellules au niveau des zones de peau lésée, soit sous la forme de cellules souches cutanées et/ou de progéniteurs épidermiques humains indifférenciés aptes à générer *in vivo* un épiderme natif et/ou tout ou partie des annexes cutanées, soit sous la forme d'épidermes reconstruits et/ou de peaux reconstruites. En particulier, les cellules pourront être injectées et/ou appliquées sur les zones à traiter, alors que les épidermes reconstruits ou peaux reconstruites seront greffés sur les zones à traiter.

L'utilisation des cellules souches cutanées et/ou des progéniteurs épidermiques indifférenciés amplifiés en présence de LIF, ou des épidermes reconstruits et/ou peaux reconstruites préparées en présence de LIF selon l'invention sera ainsi notamment destinée à traiter les brûlures.
L'utilisation des cellules souches cutanées et/ou des progéniteurs épidermiques indifférenciés amplifiés en présence de LIF, ou des épidermes reconstruits et/ou peaux reconstruites préparées en présence de LIF selon l'invention sera ainsi notamment destinée au traitement des lésions de la peau liées à une excision cutanée, en particulier d'origine chirurgicale (ex : nevus, tatouage).

Le traitement des lésions de la peau associées à une maladie génétique pourra notamment consister, selon un premier mode, en :
- la préparation d'une population de cellules souches cutanées, et/ou de progéniteurs épidermiques indifférenciés à partir d'un explant cutané de sujet atteint d'une maladie génétique affectant la peau ;
- le traitement *ex vivo* desdites cellules pour restorer la fonction défective associée à la maladie génétique, notamment par transfert du gène non muté à l'aide d'un vecteur rétroviral;
- la ré-implantation desdites cellules traitées au niveau des zones de peau lésée, soit sous la forme de cellules souches cutanées et/ou de progéniteurs épidermiques indifférenciés aptes à générer *in vivo* un épiderme natif et/ou tout ou partie des annexes cutanées, soit sous la forme d'épidermes reconstruits et/ou de peaux reconstruites. En particulier, les cellules pourront être injectées et/ou appliquées sur les zones à traiter, alors que les épidermes reconstruits ou peaux reconstruites seront greffés sur les zones à traiter.

Selon une alternative, le traitement des lésions de la peau associées à une maladie génétique pourra consister en :
- la préparation d'une population de cellules souches cutanées et/ou de progéniteurs épidermiques humains indifférenciés à partir d'un explant cutané de sujet sain (cellules hétérologues) ;
- la multiplication de ladite population de cellules souches cutanées et/ou de progéniteurs épidermiques humains indifférenciés en présence de LIF;
- l'implantation desdites cellules au niveau des zones de peau lésée, soit sous la forme de cellules souches cutanées et/ou de progéniteurs épidermiques humains indifférenciés aptes à générer *in vivo* un épiderme natif et/ou tout ou partie des annexes cutanées, soit sous la forme d'épidermes reconstruits et/ou d e peaux reconstruites. En particulier, les cellules pourront être injectées et/ou appliquées sur les zones à traiter, alors que les épidermes reconstruits ou peaux reconstruites seront greffés sur les zones à traiter.

L'utilisation des cellules souches cutanées et/ou des progéniteurs épidermiques indifférenciés amplifiés en présence de LIF, ou des épidermes reconstruits et/ou des peaux reconstruites en présence de LIF, sera ainsi destinée à traiter des lésions de la peau associées à des maladies génétiques telles que par exemple les épidermolyses bulbeuses, *Xeroderma pigmentosum,* les ichtyoses lamellaires et les ichtyoses associées au chromosome X.

L'utilisation des cellules souches cutanées et/ou de progéniteurs épidermiques indifférenciés préparés à partir un expiant cutané de sujet atteint d'une maladie génétique et cultivés en présence de LIF pourra également servir à la mise en place de modèles *in vitro* d'étude de l'efficacité de protocoles de transfert de gènes.
Il s'agira notamment d'utiliser lesdites cellules pour évaluer la fréquence de correction génétique (pourcentage de cellules présentant une insertion du transgène), et/ou pour évaluer et quantifier l'efficacité de correction fonctionnelle (par exemple, étude de la correction de la fonction initialement déficiente dans des cultures en monocouches et/ou des épiderme et/ou des peaux reconstruites).

L'invention sera maintenant illustrée par les exemples non limitatifs suivants.

### FIGURES

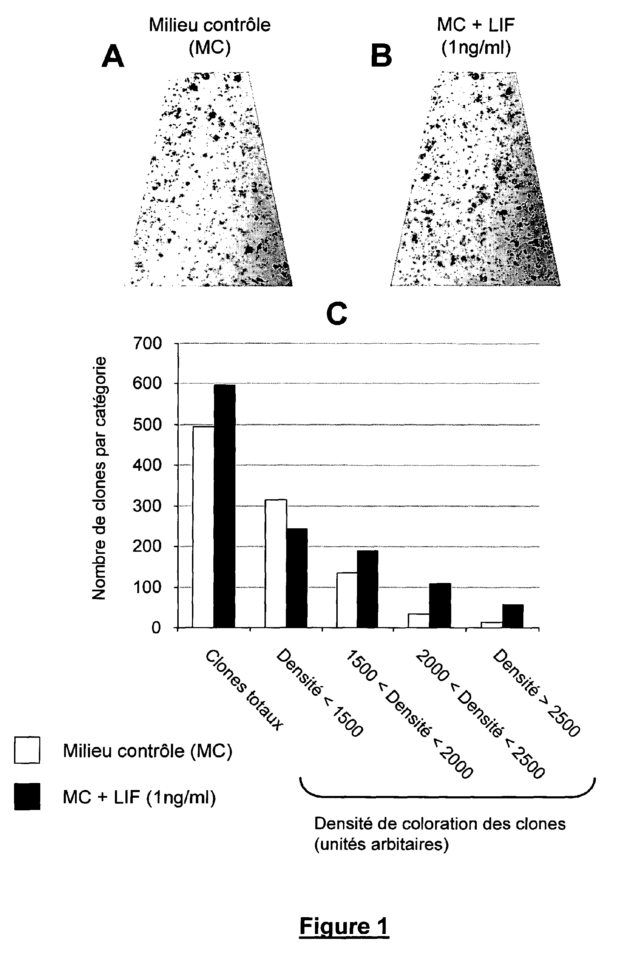

La **Figure 1** présente une analyse de l'effet du LIF sur la croissance clonogénique des cellules souches et/ou des progéniteurs épidermiques. Clones cellulaires obtenus à partir de cellules au passage 1 cultivées pendant 8 jours en absence de LIF exogène, puis fixés et colorés **(A) ;** Clones cellulaires obtenus à partir de kératinocytes au passage 1 cultivés pendant 8 jours en présence de LIF exogène à 1ng/ml, puis fixés et colorés **(B)** ; analyse semi-quantitative de la densité des clones obtenus en condition contrôle et en présence de LIF exogène **(C).**

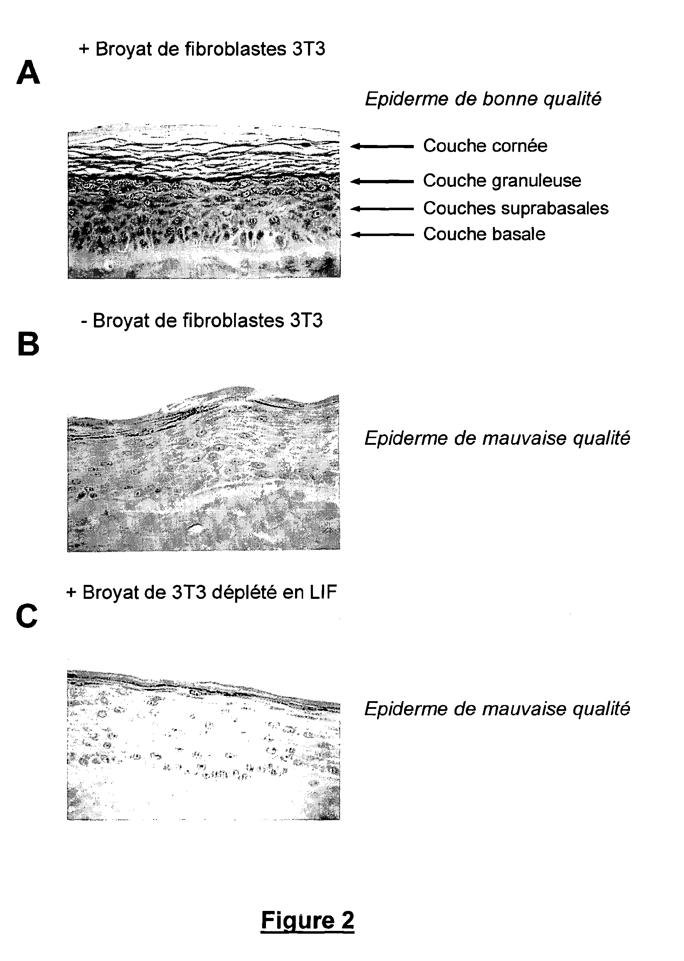

La Figure 2 présente des analyses histologiques d'un épiderme reconstruit obtenu en présence ou non d'un broyat de fibroblastes 3T3 déplété ou non en LIF. Coupe histologique d'un épiderme reconstruit obtenu en présence de broyat de 3T3 non déplété en LIF (contrôle positif) **(A) ;** Coupe histologique d'un épiderme reconstruit obtenu en absence de broyat de 3T3 (contrôle négatif) **(B) ;** Coupe histologique d'un épiderme reconstruit obtenu en présence de broyat de 3T3 déplété en LIF (condition expérimentale) (**C**).

### EXEMPLES

### Exemple 1 : Croissance des cellules souches et/ou progéniteurs épidermiques indifférenciés optimisée en présence de LIF.

Des kératinocytes ont été isolés à partir d'un prélèvement cutané adulte (plastie mammaire).

Après élimination du tissu sous-cutané à l'aide d'un scalpel, le prélèvement cutané est découpé en fragments d'environ 5mm x 5mm, puis décontaminé par un traitement antibiotique [Gentamycine (Life Technologies), 3 bains successifs de 10 minutes dans du milieu de culture DMEM (Life Technologies)]. Pour permettre la séparation du derme de l'épiderme, le prélèvement est ensuite soumis à un traitement protéolytique [dispase (Boehringer, Roche Diagnostics) + trypsine (Gibco, Invitrogen) 1 nuit à 4°C]. L'épiderme est ensuite séparé du derme par dissection. Les fragments d'épiderme séparés du tissu dermique sont placés dans une solution de trypsine 0,05%-EDTA 0,02% (Gibco, Invitrogen) (15 minutes à 37°C). La préparation est agitée périodiquement pour favoriser la dissociation des cellules. L'effet de la trypsine est ensuite neutralisé par l'ajout d'un milieu de culture contenant 10% sérum (DMEM+10%sérum). Après neutralisation de la trypsine (DMEM+10%sérum), la préparation cellulaire est homogénéisée mécaniquement (pipettages), puis filtrée. La suspension cellulaire est lavées, puis remises en suspension dans du milieu KGM (Clonetics). Les cellules en suspension sont comptées au microscope à l'aide d'une cellule de Malassez. La viabilité des échantillons est estimée par la méthode d'exclusion au bleu Trypan (Life Technologies).

Une étape d'enrichissement en cellules souches et/ou en progéniteurs épidermiques indifférenciés a été réalisée par une étape d'adhésion rapide sur un substrat de collagène de type I (sélection d'une population à forte capacité d'adhésion, Adh⁺⁺⁺).

Les cellules souches et/ou progéniteurs épidermiques indifférenciés peuvent en effet être séparées des kératinocytes plus matures sur la base de leur propriété d'adhésion rapide. Cette étape permet un pré-enrichissement de la préparation en cellules souches et/ou progéniteurs épidermiques indifférenciés.

La méthode d'enrichissement par adhésion sur collagène est décrite dans la demande WO 03/038073 et la publication scientifique (Fortunel *et al*., *J Cell Sci,* 116: 4043-4052, 2003).

La suspension cellulaire est placée dans des flacons de culture « recouverts » avec du collagène de type I [l'adsorption du collagène sur le support d'adhésion est réalisée par dépôt d'une solution liquide de collagène I (Sigma Chemical) diluée d'un facteur 2 dans du PBS, pendant au moins 45 minutes, puis séchage après élimination du surplus], à une densité de 150 000 à 200 000 cellules/cm². Après 12 à 15 minutes, les k ératinocytes n'ayant pas adhéré sont éliminés par lavage en tampon PBS. Les cellules adhérentes, dites Adh⁺⁺⁺, ainsi sélectionnées sont détachées du support par une trypsination douce (trypsine 0,05%-EDTA 0,02% (Gibco, Invitrogen) pendant 3 à 5 minutes à 37°C). Après neutralisation de la trypsine (DMEM+10%sérum), les cellules sont récupérées, lavées, puis remises en suspension dans du milieu de culture (dans le cas présent, milieu KGM). La fraction composée des cellules adhérentes, sélectionnée par cette méthode, représente environ 5 à 10% des kératinocytes totaux de l'épiderme.

Les cultures, initiées à partir de cellules Adh⁺⁺⁺, ont été réalisées en milieu de culture semi-défini (KGM Bullet Kit, Clonetics, Cambrex Bio Science Inc.), en absence de fibroblastes nourriciers, et à faible densité (ensemencements à 2400 cellules/cm²) afin d'obtenir des clones cellulaires isolés quantifiables. Au premier repiquage (passage 1), les cultures ont été divisées en 2 lots : 1) condition identique à celle décrite ci-dessus ; 2) addition de LIF à une concentration de 1ng/ml (LIF recombinant humain, Chemicon International Inc.). Après 8 jours de culture, les cultures ont été fixées (éthanol 70%) et colorées (éosine et Giemsa) afin d'analyser les caractéristiques des clones cellulaires obtenus dans ces 2 conditions de culture **(Figure 1).**

L'analyse comparative de cultures effectuées en absence ou en présence de LIF montre que ce facteur permet d'optimiser la croissance des cellules souches et/ou progéniteurs épidermiques indifférenciés issus d'une culture de cellules Adh⁺⁺⁺.

L'observation macroscopique indique une augmentation du nombre de clones cellulaires de coloration dense de grande taille **(Figure 1 A,B).** L'observation microscopique indique que ces clones dont le développement est favorisé sont essentiellement constitués de cellules de petite taille présentant des caractéristiques morphologiques associées à l'état indifférencié des cellules souches et/ou des progéniteurs épidermiques, critère traduisant une « jeunesse » plus importante des cultures traitées par le LIF. L'augmentation du nombre de clones denses de grande taille en réponse au LIF est confirmée par analyse d'image informatisée, comme représenté à l'histogramme présenté **Figure 1C**.

L'utilisation du LIF dans un système de culture permet donc de favoriser la multiplication d'une population de cellules souches épidermiques et/ou de progéniteurs épidermiques humains indifférenciés.

### Exemple 2 : Effet positif du LIF sur la reconstitution épidermique.

Nous avons observé qu'un broyat de fibroblastes 3T3 favorise la reconstitution épidermique à partir de cellules souches et/ou de progéniteurs épidermiques cultivées *in vitro* et congelés. L'épiderme reconstruit est de bonne qualité : il présente une organisation cellulaire et une stratification proche de celles d'un épiderme natif **(Figure 2A**).

Afin de tester l'hypothèse d'une implication du LIF dans cette propriété, une déplétion du LIF par immuno-précipitation a été réalisée, puis l'activité du broyat exempt de LIF a été comparée à celle du broyat non déplété.

La déplétion du broyat de 3T3 en LIF est réalisée à l'aide d'un anticorps polyclonal produit chez le lapin (Ac anti-LIF, Santa Cruz, réf. SC-20087). Le broyat de 3T3 est incubé avec l'Ac anti-LIF en excès à 4°C pendant plusieurs heures afin d'assurer une bonne fixation du LIF sur l'Ac. L'Ac libre et lié au LIF est ensuite 'trappé' par ajout de billes de sépharose sur lesquelles sont adsorbées des protéines G (les protéines G ont une forte affinité pour les domaines Fc des Ac). Une centrifugation permet séparer les billes sur lesquelles le LIF est retenu du broyat déplété en LIF.

La capacité des cellules souches épidermiques issues d'une banque congelée (isolées à partir d'un prélèvement cutané mammaire) à générer un épiderme reconstruit a été évaluée en présence de broyat de fibroblastes 3T3 non déplété en LIF (témoin positif), en absence de broyat de 3T3 (témoin négatif), et en présence de broyat de 3T3 déplété en LIF (condition expérimentale). Les caractéristiques histologiques des épidermes reconstruits obtenus dans chaque condition ont été comparées sur des coupes fixées et colorées (**Figure 2**).

Alors que les cultures organotypiques supplémentées en broyat de fibroblastes 3T3 (non déplété en LIF) permettent l'obtention d'épidermes reconstruits de bonne qualité (**Figure 2A**), c'est-à-dire une organisation cellulaire et une stratification proches de celles d'un épiderme natif, les cultures réalisées en absence de broyat ne permettent d'obtenir que des épidermes ne possédant pas les caractéristiques histologiques requises **(Figure 2B).**

Les épidermes obtenus en absence de broyat de 3T3 présentent notamment les anomalies suivantes :
- Vacuoles et espaces intercellulaires importants ;
- Mauvaise stratification ;
- Couche basale pauvre en cellules ;
- Cellules suprabasales très étirées ;
- Mauvaise orientation des cellules basales (parallèles à la matrice) ;
- Peu ou pas de couche granuleuse ;
- Couche cornée de faible épaisseur.

Les cellules de la condition expérimentale, cultivées en présence de broyat de 3T3 déplété en LIF, se comportent d'une manière similaire à celles cultivées en absence de broyat **(Figure 2C),** ce qui indique que la déplétion du LIF entraîne une perte des propriétés bénéfiques du broyat sur la reconstruction épidermique.

Ainsi , l'utilisation de LIF, outre sa capacité à favoriser la multiplication desdites cellules dans un état indifférencié comme montré à l'exemple 1, permet de maintenir le potentiel organogénique desdites cellules, c'est à dire leur capacité à générer un épiderme reconstruit de qualité, présentant des caractéristiques proches de celles de l'épiderme natif.

### Exemple 3 : Préparation d'un équivalent d'épiderme et/ou de peau.

Sauf indication contraire, l'ensemble des milieux et tampons utilisés dans les exemples sont décrits dans Bell *et al*., 1979 *(Proc Natl Acad Sci USA.* 76: 1274-1278, 1979), Asselineau et Prunieras, 1984 *(Br J Dermatol*. 111: 219-222, 1984) ou Asselineau *et al*., 1987, (Models in dermato., vol.III, Ed. Lowe & Maibach, 1-7, 1987).

Les supports ou équivalents de derme sont préparés comme décrits dans Asselineau *et al*., 1985 et 1987 *(Exp. Cell. Res.* 159: 536-539, 1985 ; Models in dermatology, vol 3 pp 1-7, 1987), dans les proportions suivantes :

| | |
|---|---|
| Milieu MEM (1,76X) | 45% |
| Sérum de Veau Foetal | 9% |
| NaOH(0.1N): | 5% |
| Acide acétique (1/1000) : | 4% |
| Collagène : | 26% |
| Fibroblastes : | 11 % |

Le collagène utilisé est du collagène de type I (solution commerciale), mais on peut également utiliser du collagène de type III ou IV. Il est extrait de queues de rat ou de peau de veau par hydrolyse acide et conservé en milieu acide à + 4°C ; il polymérise naturellement par réchauffement à 37°C et par diminution de l'acidité (augmentation du PH). Le collagène est préalablement dialysé contre des bains successifs d'eau + Acide acétique.

Le protocole est le suivant : dans un tube Falcon stérile, on introduit le milieu MEM 1,76 X en présence d'additifs (Glutamine 1%, Acides a minés non essentiels 1 %, Pyruvate de sodium 1%, Fungizone 1% et Pénicilline/Streptomycine 1%), le sérum de veau foetal, la soude NaOH 0,1 N. On a joute a lors les fibroblastes isolés à partir d'explants de peau humaine à la concentration de 1,4x 10⁵ cellules pour 1 ml de milieu de culture.
On ajoute alors lentement, contre la paroi du tube de façon à observer l'apparition d'un nuage blanchâtre, un mélange volume/volume de collagène dans de l'acide acétique dilué au 1/1000.
L'ensemble est alors mélangé avec précaution et réparti dans les puits d'une plaque de culture de 12 puits (type Costar référence 3512) à raison de 0.5ml de mélange par cm². La plaque de culture est alors placée dans un incubateur à 37°C avec 5% de CO₂.

La deuxième étape consiste à ensemencer une préparation de kératinocytes en présence de LIF ou des cellules souches cutanées et/ou des progéniteurs épidermiques indifférenciés amplifiées dans un milieu contenant du LIF sur ledit support.
Selon un mode particulier, le milieu dans lequel se trouve l'équivalent de derme est supplémenté en LIF, préalablement à l'étape d'ensemencement.
La culture peut ensuite être maintenue immergée dans un milieu nutritif 3F qui peut être par exemple le milieu décrit par Rheinwald et Green, (Cell. 6: 317-330, 1975) milieu qui permet la prolifération des kératinocytes.
Après un temps d'incubation de 3 à 15 jours, préférentiellement de 7 à 9 jours, l'équivalent de peau est maintenu à l'interface air/liquide par exemple par dépôt sur une grille métallique. Le liquide est alors préférentiellement constitué du même milieu nutritif que le précédent.

L'incubation se poursuit ensuite jusqu'à obtention d'un équivalent de peau présentant les caractéristiques d'une peau, à savoir le support sur lequel se trouve un équivalent d'épiderme présentant les quatre types de couches cellulaires classiques, à savoir les couches basale, suprabasale, granuleuse et cornée.
Ainsi, l'incubation se poursuit pendant une durée comprise entre 5 et 30 jours, préférentiellement entre 7 et 10 jours.

Le modèle de peau reconstruite ainsi réalisé comprend deux entités, le support et l'équivalent d'épiderme, qu'il est possible de séparer physiquement l'un de l'autre.

## Revendications

1. Utilisation d'une quantité efficace d'un composé choisi parmi le LIF, un analogue du LIF, un mimétique du LIF, un produit capable de stimuler l'expression endogène du LIF et leurs mélanges pour (i) favoriser la multiplication *in vitro* d'une population de cellules souches cutanées et/ou de progéniteurs épidermiques humains en les maintenant dans un état indifférencié et/ou pour (ii) maintenir et/ou augmenter leur capacité à générer un épithélium pluristratifié.

2. Utilisation d'une quantité efficace d'un composé choisi parmi le LIF, un analogue du LIF, un mimétique du LIF, un produit capable de stimuler l'expression endogène du LIF et leurs mélanges selon la revendication 1 dans un procédé destiné à l'obtention de banques ou de cultures de cellules souches cutanées et/ou de progéniteurs épidermiques humains indifférenciés.

3. Utilisation d'une quantité efficace d'un composé choi si parmi le LIF, un analogue du LIF, un mimétique du LIF, un produit capable de stimuler l'expression endogène du LIF et leurs mélanges selon la revendication 1 dans un procédé destiné à l'obtention d'épidermes reconstruits et/ou des peaux reconstruites.

4. Utilisation selon l'une quelconque des revendications 1 à 3, **caractérisée en ce que** le LIF est apporté sous la forme d'un LIF purifié, d'un LIF recombinant, d'un broyat ou d'un extrait cellulaire contenant du LIF, d'un surnageant de culture de cellules contenant du LIF ou leurs mélanges.

5. Utilisation selon l'une quelconque des revendications 1 à 3, **caractérisée en ce que** l'analogue du LIF est choisi parmi un polypeptide LIF modifié ou un fragment de polypeptide LIF ayant une activité LIF sur les cellules souches cutanées et/ou les progéniteurs épidermiques, capable (i) de favoriser la multiplication *in vitro* des cellules souches cutanées et/ou de progéniteurs épidermiques indifférenciés et (ii) de maintenir et/ou augmenter leur capacité à générer un épiderme pluristratifié.

6. Utilisation selon l'une quelconque des revendications 1 à 3, **caractérisée en ce que** le mimétique du LIF est choisi parmi tout agoniste du récepteur du LIF (LIF-R), tout actif ou extrait cellulaire capable d'activer la gp130 et/ou les voies de signalisation des Jak/Stat et Ras/Map kinases, en particulier l'expression et/ou l'activité de Stat3.

7. Procédé d'obtention d'une banque ou d'une culture de cellules souches cutanées et/ou de progéniteurs épidermiques humains indifférenciés, comprenant au moins une étape de multiplication d'une préparation de kératinocytes humains dans un milieu de culture contenant une quantité efficace d'un composé choisi parmi le LIF, un analogue du LIF, un mimétique du LIF, un produit capable de stimuler l'expression du LIF endogène et leurs mélanges.

8. Procédé selon la revendication 7, **caractérisé en ce que** la préparation de kératinocytes humains est préalablement enrichie en cellules souches cutanées et/ou en progéniteurs épidermiques humains indifférenciés, par adhésion rapide sur un substrat de collagène de type I et récupération des cellules ayant adhéré.

9. Procédé selon la revendication 8, **caractérisé en ce que** la récupération desdites cellules est réalisée par décollement en présence de trypsine 0.05% et EDTA 0.02%.

10. Procédé selon l'une quelconque des revendications 7 à 9, **caractérisé en ce que** la quantité efficace d'un composé choisi parmi le LIF, un analogue du LIF, un mimétique du LIF, un produit capable de stimuler l'expression du LIF endogène et leurs mélanges présente dans le milieu de multiplication est comprise entre 0.01pg/ml et 1 mg/ml de milieu de culture, préférentiellement entre 0.1ng/ml et 100ng/ml de milieu de culture.

11. Procédé selon l'une quelconque des revendications 7 à 10, **caractérisé en ce qu'**il comprend en outre une étape de cryo-conservation desdites cellules multipliées en présence d'un composé choisi parmi le LIF, un analogue du LIF, un mimétique du LIF, un produit capable de stimuler l'expression du LIF endogène et leurs mélanges.

12. Kit de production d'une banque ou d'une culture de cellules souches cutanées et/ou de progéniteurs épidermiques humains indifférenciés comprenant (i) une préparation de kératinocytes humains et (ii) un milieu de culture de cellules kératinocytaires humaines additionné d'une quantité efficace d'un composé choisi parmi le LIF, un analogue du LIF,un mimétique du LIF, un produit capable de stimuler l'expression du LIF endogène et leurs mélanges comprise entre 0.01pg/ml et 1mg/ml de milieu de culture, préférentiellement entre 0.1ng/ml et 100ng/ml de milieu de culture.

13. Procédé de préparation d'épidermes reconstruits et/ou de peaux reconstruites comprenant :
a) une étape de préparation d'un support ou d'un équivalent de derme; et
b) une étape d'ensemencement d'une population de k ératinocytes humains sur ledit support ;
**caractérisé en ce qu'**une quantité efficace d'un composé choisi parmi le LIF, un analogue du LIF,un mimétique du LIF, un produit capable de stimuler l'expression du LIF endogène et leurs mélanges est ajoutée dans le milieu, à l'une des étapes a) ou b), ou les deux.

14. Procédé de préparation d'épidermes reconstruits et/ou de peaux reconstruites comprenant :
a) une étape de préparation d'un support ou d'un équivalent de derme ; et
b) une étape d'ensemencement d'une population de cellules souches cutanées et/ou de progéniteurs épidermiques humains indifférenciés amplifiés en présence d'un composé choisi parmi le LIF, un analogue du LIF,un mimétique du LIF, un produit capable de stimuler l'expression du LIF endogène et leurs mélanges sur ledit support.

15. Kit de production d'un épiderme ou d'une peau reconstruite comprenant (i) un support de derme, (ii) une préparation de kératinocytes humains et (iii) un milieu de culture contenant une quantité efficace d'un composé choisi parmi le LIF, un analogue du LIF, un mimétique du LIF, un produit capable de stimuler l'expression du LIF endogène et leurs mélanges, comprise entre 0.01pg/ml et 1mg/ml de milieu de culture, préférentiellement entre 0.1ng/ml et 100ng/ml de milieu de culture.

16. Utilisation des cellules souches cutanées et/ou de progéniteurs épidermiques humains indifférenciés amplifiés en présence d'un composé choisi parmi le LIF, un analogue du LIF, un mimétique du LIF, un produit capable de stimuler l'expression du LIF endogène et leurs mélanges dans un procédé de criblage et/ou d'évaluation d'actifs susceptibles de moduler la mutiplication et/ou la différenciation de cellules souches cutanées et/ou de progéniteurs épidermiques humains indifférenciés en kératinocytes matures, ou en tout autre type cellulaire entrant dans la constitution des annexes cutanées, comprenant :
- une mise en culture de cellules souches cutanées et/ou de progéniteurs épidermiques humains indifférenciés amplifiés en présence d'un composé choisi parmi le LIF, un analogue du LIF,un mimétique du LIF, un produit capable de stimuler l'expression du LIF endogène et leurs mélanges dans des conditions permettant leur multiplication et/ou leur différenciation en kératinocytes matures ou en tout autre type cellulaire entrant dans la constitution des annexes cutanées, et en présence d'un actif à tester ;
- la comparaison de la multiplication et/ou de la différenciation des cellules en présence de l'actif avec la multiplication et/ou la différenciation des cellules en l'absence dudit actif.

17. Utilisation des cellules souches cutanées et/ou de progéniteurs épidermiques humains indifférenciés amplifiés en présence d'un composé choisi parmi le LIF, un analogue du LIF,un mimétique du LIF, un produit capable de stimuler l'expression du LIF endogène et leurs mélanges dans un procédé de criblage et/ou d'évaluation d'actifs susceptibles de moduler la capacité des cellules souches cutanées et/ou des progéniteurs épidermiques à générer un épiderme pluristratifié et/ou tout ou partie des annexes cutanées , comprenant :
- une mise en culture de cellules souches cutanées et/ou de progéniteurs épidermiques humains indifférenciés amplifiés en présence d'un composé choisi parmi le LIF, un analogue du LIF,un mimétique du LIF, un produit capable de stimuler l'expression du LIF endogène et leurs mélanges, dans des conditions leur permettant de générer un épiderme pluristratifié et/ou tout ou partie des annexes cutanées, en présence d'un actif à tester ;
- la comparaison de la capacité des cellules à générer un épiderme pluristratifié et/ou tout ou partie des annexes cutanées en présence de l'actif avec la capacité des cellules à générer un épiderme pluristratifié et/ou tout ou partie des annexes cutanées en l'absence dudit actif.

18. Utilisation d'une quantité efficace d'un composé choisi parmi le LIF, un analogue du LIF,un mimétique du LIF, un produit capable de stimuler l'expression du LIF endogène et leurs mélanges pour la préparation de cellules souches cutanées et/ou de progéniteurs épidermiques humains indifférenciés, ou d'épidermes reconstruits et/ou de peaux reconstruites selon le procédé de l'une des revendications 13 ou 14, destinés à traiter des lésions de la peau.

19. Utilisation selon la revendication 18, **caractérisée en ce que** les cellules souches cutanées et/ou progéniteurs épidermiques indifférenciés, ou les épidermes reconstruits et/ou les peaux reconstruites, sont destinés à traiter les brûlures.

20. Utilisation selon la revendication 18, **caractérisée en ce que** les cellules souches cutanées e t/ou progéniteurs épidermiques humains indifférenciés, ou les épidermes reconstruits et/ou les peaux reconstruites selon le procédé de l'une des revendications 13 ou 14, sont destinés à traiter des lésions de la peau dues à une excision cutanée.

21. Utilisation selon la revendication 18, **caractérisée en ce que** les cellules souches cutanées et/ou progéniteurs épidermiques humains indifférenciés ou les épidermes reconstruits et/ou les peaux reconstruites sont destinés à traiter des lésions de la peau associées à une maladie génétique.

22. Utilisation selon la revendication 21, **caractérisée en ce que** les cellules souches cutanées et/ou progéniteurs épidermiques humains indifférenciés sont issus de sujets atteints d'une maladie génétique affectant la peau.

23. Utilisation selon l'une des revendications 21 ou 22, **caractérisée en ce que** la maladie génétique affectant la peau est choisie parmi les épidermolyses bulbeuses, *Xeroderma pigmentosum,* les ichtyoses lamellaires ou les ichtyoses associées au chromosome X.
